# EUROPEAN PATENT APPLICATION

(11) **EP 4 316 507 A1**
(43) Date of publication of application: **07.02.2024**
(21) Application number: 22776151.7
(22) Date of filing: 25.03.2022
(51) Int. Cl.: A61K 38/44, A61K 38/16, A61K 38/19, A61P 35/00, A23L 33/17, A23L 33/195, C12N 15/63, C12N 9/02, C07K 14/55, C07K 14/54

(54) **COMPOSITION FOR PREVENTING OR TREATING CANCER COMPRISING DUAL EXPRESSION VECTOR FOR SIMULTANEOUSLY EXPRESSING PROTEIN PRESENT IN CELL AND PROTEIN SECRETED OUT OF CELL**

(30) Priority: 26.03.2021 KR 20210039575
(71) Applicant: YD Life Science Co., Ltd., Seongnam-si, Gyeonggi-do 13207 (KR); Korea Research Institute of Bioscience and Biotechnology, Daejeon 34141 (KR)
(72) Inventor: RYU, Incheol, Seongnam-si Gyeonggi-do 13207 (KR); JEONG, Daeun, Seongnam-si Gyeonggi-do 13207 (KR); AN, Sihyeon, Seongnam-si Gyeonggi-do 13207 (KR); KIM, Seong Heon, Seongnam-si Gyeonggi-do 13207 (KR); KIM, Yunseon, Seongnam-si Gyeonggi-do 13207 (KR); RYOO, Kanghyun, Seongnam-si Gyeonggi-do 13207 (KR); LEE, Hyunseung, Seongnam-si Gyeonggi-do 13207 (KR); YOON, Dong-Oh, Seongnam-si Gyeonggi-do 13207 (KR); LEE, Myoungwoo, Seongnam-si Gyeonggi-do 13207 (KR); WON, Misun, Daejeon 34141 (KR); IM, Joo-Young, Daejeon 34141 (KR)
(74) Representative: Nederlandsch Octrooibureau
(86) International application number: PCT/KR2022/004224
(87) International publication number: WO 2022/203451

(57) **Abstract**

The present invention relates to a composition for preventing or treating cancer, comprising a dual expression vector, and more specifically, to a method for effectively treating cancer by simultaneously inducing the actions of cancer metabolism drugs and cancer immunotherapy drugs by using a dual expression vector for simultaneously expressing an HIF-1α inhibitor and anticancer cytokines.

## Description

### [Technical Field]

The present invention relates to a composition for preventing or treating cancer, including a dual expression vector, and more specifically, to a method for effectively treating cancer by simultaneously inducing the actions of cancer metabolism drugs and cancer immunotherapy drugs by using a dual expression vector for simultaneously expressing an HIF-1α inhibitor in cells and anticancer cytokines that can be secreted out of cells.

### [Background Art]

Despite humanity's unceasing efforts over the last several decades, cancer remains an intractable disease and one of the main unmet medical needs. Various biochemical mechanisms related to the onset of cancer have been elucidated, and even though numerous anticancer drugs have been developed based on such knowledge, no effective treatment method has been proposed to date. Recently, due to the development and spread of early diagnosis methods, a treatment effect close to a complete cure is shown when cancer is diagnosed at an early stage, but the number of cancer patients is steadily increasing due to socio-environmental factors, and in many cases, the cancer has already progressed considerably by the time patients start to feel subjective symptoms, so cancer cure and survival rates are still not high. In fact, according to a 2015 report by the World Health Organization, cancer is the first and second leading cause of death before the age of 70 in 91 of the world's 172 countries.

Cancer is caused by the accumulation of mutated genes in cells due to genetic and environmental factors, resulting in uncontrolled cell proliferation and the formation of abnormal tissues. In particular, the mutation that has recently become the biggest problem occurs in exons 18, 19, 20, or 21 of EGFR, and the like, such as nucleotide deletion in exon 19, the L858R mutation in exon 21, and the like. Further, G12C mutations in KRAS, V900E mutations in BRAF, mutations caused by chromosomal rearrangement of ROS1, and the like are also pointed out as one of the major causes.

As a treatment method for cancer, not only surgical procedures such as surgery and radiation, but also treatment using anticancer drugs are often performed. For example, since first-generation chemical anticancer drugs such as the vinca alkaloid series (vincristine, vinblastine, vinorelbine), the taxol series (paclitaxel, docetaxel), the platinum series (cisplatin, carboplatin), doxorubicin, etoposide, gemcitabine, and pemetrexed have been used as anticancer drugs that suppress cell division, and have a large effect not only on cancer cells but also on normal cells, they cause serious side effects such as general fatigue, alopecia, nausea, vomiting, loss of appetite, diarrhea, oral inflammation, cytopenia and damage to specific organs.

To overcome these disadvantages, targeted anticancer drugs developed since the mid-1990s have been developed for the purpose of treating cancer by targeting cancer-related genes such as specific mutated genes. Examples of representative targeted anticancer drugs include gefitinib, erlotinib, afatinib, osimertinib, crizotinib, ceritinib, alectinib, brigatinib, dabrafenib, and the like, which target EGFR mutations, ALK mutations, BRAF mutations, and the like. Although these targeted anticancer drugs have the advantage of lowering side effects due to lower toxicity than chemical anticancer drugs, they have problems in which not only the cancer treatment effect deteriorates, but also the subsequent recurrence rate of cancer is increased because it is known that resistant cancer cells capable of avoiding the mechanism of action of targeted anticancer drugs frequently emerge within 8 to 14 months after administration. For example, the emergence of drug-resistant EGFR T790M mutations to EGFR inhibitors may be cited as representative examples.

Thereafter, an immunotherapy drug that helps to induce the death of cancer cells in the body by activating the immune system suppressed in cancer tissue has been proposed as an alternative to overcome the limitations of existing anticancer drugs. Theoretically, cancer immunotherapy drugs can be applied to most cancers regardless of specific gene mutations, and it was expected that there would be fewer side effects than previous anticancer drugs. Contrary to expectations, however, the therapeutic effect of the cancer immunotherapy drug was not significant. For example, a cancer immunotherapy drug Keytruda targeting PD-1/PD-L1, an immune checkpoint inhibitor, had very low reactivity when applied to treatment. In particular, in the case of non-small cell lung cancer, only 20-30% of patients showed therapeutic effects, and the remaining 70-80% of patients showed little response. Therefore, there is an urgent need for developing effective anticancer drugs for various cancers.

Recently, attempts have been made to develop metabolic anticancer drugs that target cancer cell-specific metabolic processes, particularly the processes of mitochondrial metabolism, autophagy activation, and random absorption of nutrients from the outside under hypoxic conditions, and they are expected to be able to overcome the high toxicity of first-generation chemical anticancer drugs, the frequent occurrence of resistance to second-generation targeted anticancer drugs, and the low response rate to third-generation cancer immunotherapy drugs. In particular, with regard to the hypoxic state in cancer tissue, hypoxia-inducible factor-1 (HIF-1) is a transcription factor induced in hypoxia, and is known as the most important molecule in regulating the adaptation of cancer cells to the hypoxic state and, in particular, the amount of HIF-1α protein is known to have a close correlation with the prognosis of cancer patients. HIF-1 is activated by cancer cell growth factor stimulation, hypoxic conditions, oncogene activation, or inactivation of tumor suppressor genes such as pVHL, and it is known that activated HIF-1 induces the expression of genes such as hexokinase 2, glucose transporter 1, erythropoietin, IGF-2, endoglin, VEGFA, MMP-2, uPAR, and MDR1 such that cancer cells become malignant by acquiring traits such as resistance to cell death (apoptosis), enhancement of angiogenesis ability, increase in cell proliferation ability, cell migration (metastasis) and invasion ability. Therefore, although many attempts have been made to treat cancer by suppressing HIF-1α, no therapeutic agents effective enough to be substantially commercialized and used have been developed (Korean Patent Publication No. 10-2015-0039264).

Therefore, based on the results of intensive studies to use HIF-1α inhibitors as effective cancer therapeutic agents, the present inventors developed an effective dual expression vector capable of modulating cancer-specific metabolism by HIF-1α inhibitors and simultaneously exhibiting an immune anticancer effect by anticancer cytokines.

### [Disclosure]

### [Technical Problem]

The present invention has been devised in order to solve the above-described problems in the related art, and it is an object of the present invention to provide a composition for preventing or treating cancer, including, as an active ingredient, a dual expression vector that simultaneously expresses an HIF-1α inhibitor in cells and an anticancer cytokine that can be secreted outside the cell.

However, the technical problems which the present invention intends to solve are not limited to the technical problems which have been mentioned above, and other technical problems, which have not been mentioned, will clearly be understood by those with ordinary skill in the art to which the present invention pertains from the following description.

### [Technical Solution]

The present invention provides a pharmaceutical composition for preventing or treating cancer, including, as an active ingredient, a recombinant vector including a polynucleotide encoding a hypoxia-inducible factor-1α inhibitor, a polynucleotide encoding a 2A peptide, and a polynucleotide encoding an anticancer cytokine.

In addition, the present invention provides a food composition for preventing or improving cancer, including, as an active ingredient, a recombinant vector including a polynucleotide encoding a hypoxia-inducible factor-1α inhibitor, a polynucleotide encoding a 2A peptide, and a polynucleotide encoding an anticancer cytokine.

In an exemplary embodiment of the present invention, the recombinant vector may be in a form in which a polynucleotide encoding a 2A peptide is inserted between a polynucleotide encoding a hypoxia-inducible factor-1α inhibitor and a polynucleotide encoding an anticancer cytokine, the recombinant vector may be in a form in which a polynucleotide encoding a hypoxia-inducible factor-1α inhibitor, a polynucleotide encoding a 2A peptide, and a polynucleotide encoding an anticancer cytokine are sequentially linked so as to be operable in this order, or in a form in which a polynucleotide encoding an anticancer cytokine, a polynucleotide encoding a 2A peptide, and a polynucleotide encoding a hypoxia-inducible factor-1α inhibitor are sequentially linked so as to be operable in this order, but is not limited thereto as long as the recombinant vector is a dual expression vector in a form in which an HIF-1α inhibitor and an anticancer cytokine can be simultaneously expressed.

In another exemplary embodiment of the present invention, the hypoxia-inducible factor-1α inhibitor may be preferably a protein capable of inhibiting the expression or activity of hypoxia-inducible factor-1α, more preferably cytochrome b5 reductase 3, the von Hippel Lindau tumor suppressor protein (pVHL), arrest-defective-1 (ARD-1), hypoxia-inducible factor (HIF) prolyl-hydroxylase, Kelch-like ECH-associated protein 1(Keap1)-NF-E2-related factor 2 (Nrf2), and the like. Alternatively, the hypoxia-inducible factor-1α inhibitor may be an antisense oligonucleotide (ASO), small interfering RNA (siRNA), microRNA (miRNA), an aptamer, an antibody, or the like, such as directly inhibit expression or activity of hypoxia-inducible factor-1α through binding. However, since the dual expression vector of the present invention acts as a mechanism to metabolically inhibit the death, migration, metastasis, proliferation, and the like of cancer cells by inhibiting hypoxia-inducible factor-1α in the body, the hypoxia-inducible factor-1α inhibitor is not limited thereto as long as the inhibitor is a protein capable of inhibiting the expression or activity of hypoxia-inducible factor-1α. The cytochrome b5 reductase 3 may be most preferably a protein including an amino acid sequence represented by SEQ ID NO: 1, but may include an amino acid sequence having a sequence homology of preferably 80% or more, more preferably 90% or more, and even more preferably 95% or more with the amino acid sequence of SEQ ID NO: 1. The % sequence homology to an amino acid sequence is confirmed by comparing a comparison region with an optimally aligned sequence, and a portion of the amino acid sequence in the comparison region may include an addition or deletion (that is, gap) compared to a reference sequence (without addition or deletion) for the optimal alignment of the sequences.

In still another exemplary embodiment of the present invention, the 2A peptide may include an amino acid sequence represented by SEQ ID NO: 5.

In still another exemplary embodiment of the present invention, the anticancer cytokine may be interferon-alpha, interferon-beta, interferon-gamma, interleukin 2, interleukin 7, interleukin 12, interleukin 15, interleukin 18, interleukin 21, interleukin 37, a granulocyte-macrophage colony-stimulating factor, or the like, and is not limited as long as the anticancer cytokine is an anticancer cytokine known to cause an immune response in a cancer cell-specific manner by activating T cells, B cells, natural killer cells (NK cells), and the like.

In yet another exemplary embodiment of the present invention, the recombinant vector may be linear DNA, plasma DNA, and a viral vector, and the viral vector may be a retrovirus, an adenovirus, an adeno-associated virus, vaccinia virus, Myxoma virus, herpes simplex virus, a lentivirus, or the like, but is not limited thereto as long as it is a type that can be used as a dual expression vector capable of simultaneously expressing an HIF-1α inhibitor and an anticancer cytokine in the body.

In still yet another exemplary embodiment of the present invention, the cancer may be preferably melanoma, small cell lung cancer, non-small cell lung cancer, glioma, liver cancer, thyroid tumors, gastric cancer, prostate cancer, breast cancer, ovarian cancer, bladder cancer, lung cancer, colorectal cancer, breast cancer, prostate cancer, glioblastoma, endometrial cancer, renal cancer, colon cancer, pancreas cancer, esophageal carcinoma, head and neck cancer, mesothelioma, sarcomas, bile duct cancer, small bowel adenocarcinoma, pediatric malignant cancer, epidermal cancer, and the like, but is not limited thereto as long as it is any cancer type that can be treated by inhibiting hypoxia-inducible factor-1α.

Furthermore, the present invention provides a method for preventing or treating cancer, the method including administering a composition including, as an active ingredient, a recombinant vector including a polynucleotide encoding a hypoxia-inducible factor-1α inhibitor, a polynucleotide encoding a 2A peptide, and a polynucleotide encoding an anticancer cytokine to a subject in need.

Further, the present invention provides a use of a composition including, as an active ingredient, a recombinant vector including a polynucleotide encoding a hypoxia-inducible factor-1α inhibitor, a polynucleotide encoding a 2A peptide, and a polynucleotide encoding an anticancer cytokine for preventing or treating cancer.

In addition, the present invention provides a use of a recombinant vector including a polynucleotide encoding a hypoxia-inducible factor-1α inhibitor, a polynucleotide encoding a 2A peptide, and a polynucleotide encoding an anticancer cytokine for producing a drug used against cancer treatment.

### [Advantageous Effects]

The dual expression vector for simultaneously expressing an HIF-1α inhibitor and an anticancer cytokine according to the present invention can improve the therapeutic effect of a metabolic anticancer drug by effectively inhibiting the proliferation, migration, metastasis, and the like of cancer due to a material capable of inhibiting HIF-1α, that is, a metabolic anticancer drug and simultaneously activating T cells, B cells, NK cells, and the like due to an anticancer cytokine, that is, an anticancer immune inhibitor, to induce a cancer-specific immune response. In addition, the dual expression vector can reduce the pain and expenses of a patient by administering one vector to reduce the frequency of administration. Furthermore, since the cancer immunotherapy drug and the metabolic anticancer drug are simultaneously expressed, side effects can be reduced and the occurrence of resistance to anticancer drugs occurring in the body can also be reduced because even a small amount has a high therapeutic effect. Therefore, it is expected that the dual expression vector of the present invention can be effectively applied to the treatment of various cancers. In addition, given that anticancer treatment should be differentiated according to the genetic cause or causative factor of the onset of cancer, since the present invention can provide various treatment options of metabolic anticancer drugs and cancer immunotherapy drugs, it is expected that the anticancer treatment rate can be remarkably enhanced.

### [Description of Drawings]

FIG. 1 is a view schematically showing the structure of a dual expression vector, a protein produced by the dual expression vector, its location, and its effect on cancer according to an exemplary embodiment of the present invention.
FIG. 2 is a schematic view of the dual expression vector according to an exemplary embodiment of the present invention.
FIG. 3 is a view showing the results of confirming whether the dual expression vector according to an exemplary embodiment of the present invention is expressed by western blotting.
FIG. 4 is a view showing the results of confirming the anticancer effect of the dual expression vector according to an exemplary embodiment of the present invention.
FIG. 5 is a view showing the results of confirming the anticancer effect of the dual expression vector injected into an adenovirus according to an exemplary embodiment of the present invention.
FIG. 6 is a view showing the results of confirming NK immune cell activation by a cytokine secreted from the dual expression vector according to an exemplary embodiment of the present invention.
FIG. 7 is a view showing the results of confirming the immune activation effect of anticancer cytokines of the dual expression vector according to an exemplary embodiment of the present invention.

### [Best Modes of the Invention]

It was confirmed that a dual expression vector of the present invention for simultaneously expressing an HIF-1α inhibitor and an anticancer cytokine can express the HIF-1α inhibitor and the anticancer cytokine, and the expressed proteins normally work and thus does not exhibit toxicity in normal cells, but can effectively treat cancer by inducing apoptosis in a cancer cell-specific manner. Therefore, it is expected that the dual expression vector of the present invention for simultaneously expressing an HIF-1α inhibitor and an anticancer cytokine, can be applied as an effective therapeutic agent for multiple cancers that have developed resistance.

In the present specification, "recombinant vector" refers to a vector capable of expressing a peptide or protein encoded by a foreign nucleic acid inserted in the vector, preferably a vector prepared so as to include an HIF-1α inhibitor gene and an anticancer cytokine gene. "Vector" refers to any medium for the introduction and/or transfer of a base in a host cell in vitro, ex vivo, or in vivo, and may be a replicon to which another DNA fragment may be bound to bring about the replication of the bound fragment, and "replicon" refers to any genetic unit (for example, a plasmid, a phage, a cosmid, a chromosome, a virus, and the like) that functions as an autonomous unit of DNA replication in vivo, that is, one which is capable of replication under its own control. The recombinant expression vector of the present invention may include preferably a promoter, which is a transcription initiation factor to which an RNA polymerase is bound, an arbitrary operator sequence for regulating transcription, a sequence encoding an appropriate mRNA ribosome binding site, a sequence regulating the termination of transcription and translation, a terminator, and the like, may additionally include more preferably a tag gene for increasing the production amount of a recombinant protein, a tag gene for maintaining the structural stability of the recombinant protein, a tag gene for easily separating the recombinant protein, a selection marker gene such as an antibiotic-resistant gene for selecting a transgenic organism, and the like, representative examples of a tag for easy separation include an Avi tag, a Calmodulin tag, a polyglutamate tag, an E tag, a FLAG tag, an HA tag, a His tag, a Myc tag, an S tag, an SBP tag, an IgG-Fc tag, a CTB tag, a Softag 1 tag, a Softag 3 tag, a Strep tag, a TC tag, a V5 tag, a VSV tag, an Xpress tag, a FLAG tag, and the like, representative examples of the selection marker gene include a gene having resistance to antibiotics such as kanamycin, G418, bleomycin, hygromycin, chloramphenicol, ampicillin, and tetracycline, and the like, representative examples of the promoter include a pEMU promoter, a MAS promoter, a histone promoter, a Clp promoter, a 35S promoter derived from cauliflower mosaic virus, a 19S RNA promoter derived from cauliflower mosaic virus, a ubiquitin protein promoter, a cytomegalovirus (CMV) promoter, a simian virus 40 (SV40) promoter, a respiratory syncytial virus (RSV) promoter, an elongation factor-1 alpha (EF-1α) promoter, and the like, representative examples of the terminator include nopaline synthase (NOS), a phaseolin terminator, an Octopine gene terminator of Agrobacterium tumefaciens, an E. coli rrnB1/B2 terminator, and the like, but the type of added gene is not limited as long as it is a type used for the preparation of existing recombinant proteins.

As used herein, "operably linked" refers to a state in which a nucleic acid expression control sequence and a nucleic acid sequence encoding a target protein or RNA are functionally linked so as to carry out a general function. For example, a promoter and a nucleic acid sequence encoding a protein or RNA may be operably linked to affect the expression of the coding sequence. An operable linkage with an expression vector may be prepared using a gene recombination technique well-known in the art, and site-specific DNA cleavage and ligation may use enzymes generally known in the art, or the like.

As used herein, "anticancer cytokine" collectively refers to a cytokine known to induce immune responses specific to cancer cells and cancer by activating T cells, B cells, NK cells, and the like in the body, and serves to kill cancer cells through autoimmunity. An example thereof may include interferon-alpha, interferon-beta, interferon-gamma, interleukin 2, interleukin 7, interleukin 12, interleukin 15, interleukin 18, interleukin 21, interleukin 37, a granulocyte-macrophage colony-stimulating factor, or the like, IL-2 is known to regulate the growth and activity of T cells, NK cells, B cells, and monocytes, and induce cancer cell-specific immune responses at high concentrations, and IL-7 is known to be involved in the survival of T cells, the development of B cells, and the proliferation and maintenance of homeostasis of these cells by acting on T cells, B cells, and the like. It is also known to enhance immune action by increasing the survival of CD56^{Bight} NK cells and the like. IL-12 (p70) is known to induce an anticancer effect by inducing the differentiation of naive T-helper cells into Th1 cells, increase the activity of cytotoxic T cells, and increase the survival of B cells. IL-15 is known to regulate the proliferation and activity of T cells and NK cells by acting on these cells and to inhibit the induction of apoptosis in these immune cells. IL-21 is known to promote the growth of NK cells, T cells, and B cells and increase the activity of these cells by acting on these cells. It is known that IL-37 is a cytokine belonging to the IL-1 family, exhibits an antitumor effect against various cancer types according to its concentration and interactions with other units, and in particular, can induce the death of cancer cells by acting on cancer cells to increase the expression of p-STAT3 and VEGF, or regulate the tumor microenvironment by inhibiting tumor angiogenesis and acting on macrophages to inhibit tumor-promoting inflammation, and the like. It is known that GM-CSF can exhibit an anticancer effect by inducing the differentiation of myeloid cells, and particularly, can induce CTL-mediated tumor lysis by promoting the maturation of dendritic cells to promote the process of presenting a tumor-specific antigen.

As used herein, "cancer" refers to a malignant solid tumor that is predicted to grow indefinitely along with various hematological cancers that originate from stem cells in hypoxic bone marrow that can expand locally by infiltration and systematically through metastasis. Although not particularly limited thereto, specific examples of cancer include adrenal cancer, bone cancer, brain cancer, breast cancer, bronchial cancer, colon and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of cancer include adenocarcinoma, adenoma, basal cell carcinoma, cervical dysplasia and epithelial cancer, Ewing's sarcoma, squamous cell carcinoma, axillary cell carcinoma, malignant brain tumors, blastomas, intestinal ganglion neuroma, hyperplastic corneal nerve cancer, islet cell carcinoma, Kaposi cancer, leiomyoma, leukemia, lymphoma, malignant carcinoid syndrome, malignant melanomas, malignant hypercalcemia, Marfanoid habitus cancer, myeloid cancer, metastatic skin cancer, mucosal neuroma, myelodysplastic syndrome, myeloma, filamentous sarcoma, neuroblastoma, osteosarcoma, osteogenic and other sarcomas, ovarian cancer, pheochromocytoma, polycythemia vera, primary brain tumors, small cell lung cancer, ulcerative and papillary squamous cell carcinoma, seminomas, soft tissue sarcoma, retinoblastoma, rhabdomyoblastoma, reticulum cell sarcoma, and Wilms' tumors. Specific examples of cancer may also include astrocytomas, gastrointestinal stromal tumors (GISTs), gliomas or glioblastomas, renal cell carcinoma (RCC), hepatocellular carcinoma (HCC), pancreatic neuroendocrine cancer, and the like.

As used herein, "prevention" refers to all actions that inhibit a disease such as cancer or delay the onset of the disease by administering the composition according to the present invention.

As used herein, "treatment" refers to all actions in which symptoms such as a cancer disease are ameliorated or advantageously changed by administering the composition according to the present invention.

As used herein, "improvement" refers to all actions that at least reduce a parameter associated with a condition to be treated, for example, the degree of symptoms. In this case, the composition of the present invention may be used simultaneously with or separately from a therapeutic drug for the prevention or amelioration of cancer.

As used herein, "subject" refers to a subject to which the composition of the present invention may be administered, and the subject is not limited.

As used herein, a pharmaceutical composition may be in the form of a capsule, a tablet, a granule, an injection, an ointment, a powder, or a beverage, and the pharmaceutical composition may be characterized in that it is intended for humans. The pharmaceutical composition of the present invention may include a pharmaceutically acceptable carrier. As the pharmaceutically acceptable carrier, in the case of oral administration, a binder, a lubricant, a disintegrant, an excipient, a solubilizing agent, a dispersing agent, a stabilizer, a suspending agent, a colorant, a flavoring agent, and the like, in the case of injection, a buffering agent, a preservative, an analgesic, a solubilizer, an isotonic agent, a stabilizer, and the like may be mixed and used, and in the case of topical administration, a base, an excipient, a lubricant, a preservative, and the like may be used. The formulation of the pharmaceutical composition of the present invention may be variously prepared by mixing the pharmaceutical composition of the present invention with the pharmaceutically acceptable carrier described above. For example, the formulation may be prepared in the form of a tablet, a troche, a capsule, an elixir, a suspension, a syrup, a wafer, and the like for oral administration, and in the case of injection, the injection may be formulated into unit dosage ampoules or in multiple dosage forms. The pharmaceutical composition of the present invention may also be used by being formulated into sugar-coated tablets, gels, pills, powders, granules, suppositories, external preparations, solutions, suspensions, sustained-release preparations, slurries, and the like. Meanwhile, as an example of suitable carriers, excipients and diluents for formulation, it is possible to use lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, or the like. Further, the pharmaceutical composition of the present invention may further include a filler, an anticoagulant, a lubricant, a wetting agent, a flavoring agent, an emulsifier, an antiseptic, and the like.

The route of administration of the pharmaceutical composition according to the present invention is not limited to the following routes, but oral or parenteral administration is preferred, and for example, oral, intravenous, intramuscular, intraarticular, intrasynovial, intraarterial, intramedullary, intrathecal, intracardiac, percutaneous, intradermal, subcutaneous, intraperitoneal, intranasal, intestinal, topical, sublingual, rectal, intracisternal, intralesional, intracranial administration, and the like are included.

The dosage of the pharmaceutical composition of the present invention may vary depending on various factors including the activity of the specific compound used, age, body weight, general health, sex, diet, time of administration, route of administration, rate of excretion, drug combination and the severity of the particular disease to be prevented or treated, and varies depending on the condition, body weight, and the degree of disease of the patient, the form of drug, the route of administration and duration, but may be appropriately selected by a person skilled in the art, and may be 0.0001 to 500 mg/kg or 0.001 to 500 mg/kg daily. The administration may be carried out once a day, or may be divided into several times. Alternatively, when a pharmaceutical composition is prepared through a viral vector based on a dual expression vector, based on a plaque formation unit (PFU), which is a basic unit according to viral infectivity, a range of 1 to 10¹⁰⁰ PFU or IFU or more may be administered in a single dose or divided into several doses. In addition, 1 to 10¹⁰⁰ or more infectious viral particles (VP or VG) may be administered in a single dose or divided into several doses according to the type of virus and the potency that can be secured. The dosage is not intended to limit the scope of the present invention in any way.

In the present specification, the food composition may be used in various foods, for example, beverages, gums, teas, vitamin complexes, and health supplements, and may be used in the form of a pill, a powder, a granule, an infusion, a tablet, a capsule, or a beverage. The food composition includes a health functional food composition. In this case, the amount of the dual expression vector in a food or a beverage may be 0.01 to 30 wt% based on the weight of the entire food in the case of the food composition of the present invention, and in the case of the health beverage composition, an amount of 0.02 to 10g, preferably 0.3 to 1 g, based on 100 mL may be added.

The food composition of the present invention has no particular limitation in an ingredient to be added in addition to the dual expression vector as an essential ingredient, and may include typical food additives used in the art, for example, natural carbohydrates, flavorants, flavoring agents, colorants, fillers, stabilizers, various nutrients, vitamins, minerals (electrolytes) and the like. As an example of the natural carbohydrates, typical sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol may be used. As an example of the flavorant, a natural flavorant (thaumatin, stevia extract (for example, rebaudioside A, glycyrrhizin and the like), and a synthetic flavorant (saccharin, aspartame and the like) may be advantageously used. As other examples of the flavoring agent, honey, D-mannitol, a maltitol solution, a krill concentrate, and the like may be used. In addition to the above, the food composition of the present invention may contain pectic acid and salts thereof, alginic acid and salts thereof, organic acids, protective colloid thickeners, pH adjusters, stabilizers, preservatives, glycerin, alcohols, carbonating agents used in carbonated drinks, and the like. These ingredients may be used either alone or in combinations thereof. The proportion of these additives is not significantly important, but is generally selected within a range of 0 to 20 parts by weight per 100 parts by weight of the composition of the present invention.

Hereinafter, preferred examples for helping the understanding of the present invention will be suggested. However, the following examples are provided only so that the present invention may be more easily understood, and the content of the present invention is not limited by the following examples.

### [Examples]

### Example 1: Construction of dual expression vector for simultaneously expressing HIF-1α inhibitor and anticancer cytokine

In order to construct a dual expression vector for simultaneously expressing a metabolic anticancer drug and a cancer immunotherapy drug, a cytochrome b5 reductase (CYB5R3, SEQ ID NO: 1) gene known to inhibit the expression of hypoxia-inducible factor-1α (HIF-1α) was used as an example of the metabolic anticancer drug. Furthermore, as an example of a cytokine, which is a cancer immunotherapy drug, interleukin 2 (IL2, SEQ ID NO: 9), interleukin 7 (IL7, SEQ ID NO: 11), interleukin 12A (IL12A, SEQ ID NO: 15), interleukin 12B (IL12B, SEQ ID NO: 13), interleukin 15 (IL15, SEQ ID NO: 17), interleukin 21 (IL21, SEQ ID NO: 19), interleukin 37 (IL37, SEQ ID NO: 21), and a granulocyte-macrophage colony-stimulating factor (GM-CSF, SEQ ID NO: 23) were used. More specifically, a multi-cloning site (MCS) in a pDE1 vector including a base sequence of SEQ ID NO: 25 was cut using an NheI restriction enzyme and a Xbal restriction enzyme, and genes encoding a CYB5R3 protein (SEQ ID NO: 1) bound with an HA epitope (SEQ ID NO: 3) at the C-terminus, a 2A peptide (porcine teschovirus-1 2A; CS peptide, SEQ ID NO: 5), and a cytokine with a FLAG peptide (SEQ ID NO: 7) bound at the C-terminus, respectively, were sequentially inserted.

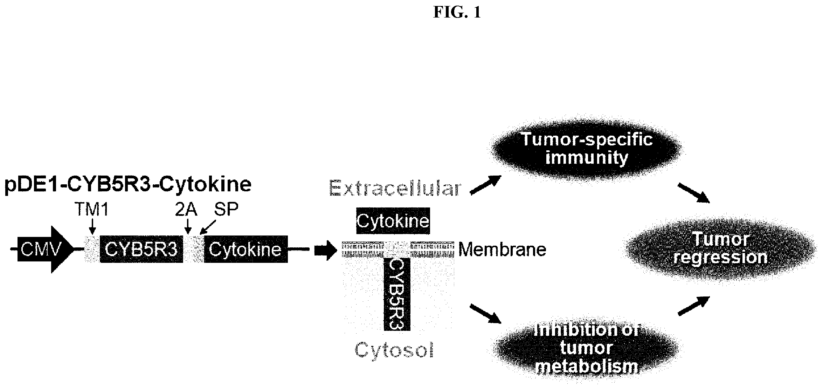

The dual expression vector thus constructed was transcribed into mRNA expressing a CYB5R3 protein bound with an HA epitope at the C-terminus-a 2A peptide-a cytokine with a FLAG peptide bound at the C-terminus, and the transcribed mRNA was translated into protein by a ribosome. In this case, by ribosome skipping, the previous sequence and the subsequent sequence before/after the 2A peptide sequence were separately translated into two proteins, the CYB5R3 protein was bound to the plasma membrane or endoplasmic reticulum (ER) membrane by the transmembrane domain 1 (TM1) included at the N-terminus of the protein to be present in the cytoplasm, and the cytokine protein was extracellularly secreted in a mature form (a final protein sequence in Table 1) according to the signal peptide (SP) sequence included in the protein. The structure of the dual expression vector of the present invention and the location of a protein prepared by the dual expression vector are schematically shown in FIG. 1.

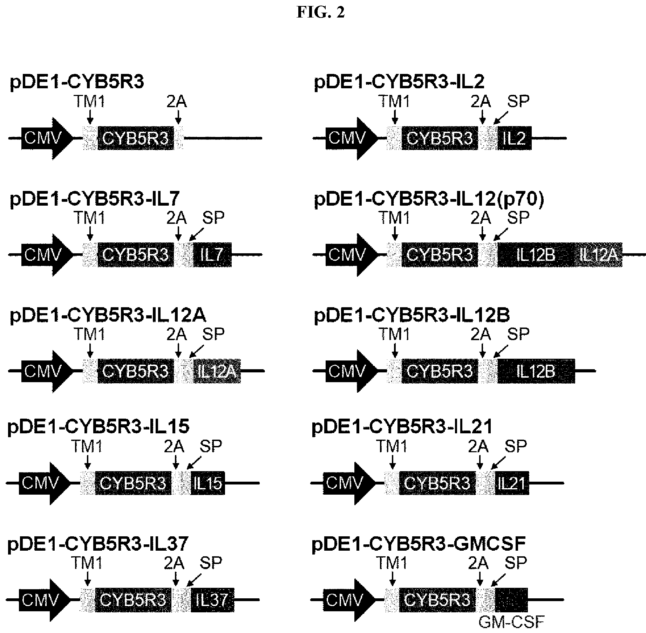

A summary of the structures and nucleotide sequences of the constructed vectors is shown in FIG. 2 and Table 1.

**[Table 1]**

| **Vector name** | **HIF-1α inhibitor gene sequence** | **HA epitope gene sequence** | **2A gene sequence** | **Cytokine gene sequence** | **FLAG gene sequence** | **Entire vector gene sequence** | **Final protein sequence** |
|---|---|---|---|---|---|---|---|
| **pDE1-CYB5R3** | SEQ ID NO: 2 | SEQ ID NO: 4 | SEQ ID NO: 6 | - | - | SEQ ID NO: 26 | SEQ ID NO: 36 |
| **pDE1-CYB5R3 -IL2** | SEQ ID NO: 2 | SEQ ID NO: 4 | SEQ ID NO: 6 | SEQ ID NO: 10 | SEQ ID NO: 8 | SEQ ID NO: 27 | SEQ ID NO: 36 / SEQ ID NO: 37 |
| **pDE1-CYB5R3 -IL7** | SEQ ID NO: 2 | SEQ ID NO: 4 | SEQ ID NO: 6 | SEQ ID NO: 12 | SEQ ID NO: 8 | SEQ ID NO: 28 | SEQ ID NO: 36 / SEQ ID NO: 38 |
| **pDE1-CYB5R3 IL12(p7 0)** | SEQ ID NO: 2 | SEQ ID NO: 4 | SEQ ID NO: 6 | SEQ ID NO: 14 + 169 to 759 nucleotides of SEQ ID NO: 16 | SEQ ID NO: 8 | SEQ ID NO: 29 | SEQ ID NO: 36 / SEQ ID NO: 39 |
| **pDE1-CYB5R3 -IL12A** | SEQ ID NO: 2 | SEQ ID NO: 4 | SEQ ID NO: 6 | SEQ ID NO: 16 | SEQ ID NO: 8 | SEQ ID NO: 30 | SEQ ID NO: 36 / SEQ ID NO: 40 |
| **pDE1-CYB5R3 -IL12B** | SEQ ID NO: 2 | SEQ ID NO: 4 | SEQ ID NO: 6 | SEQ ID NO: 14 | SEQ ID NO: 8 | SEQ ID NO: 31 | SEQ ID NO: 36 / SEQ ID NO: 41 |
| **pDE1-CYB5R3 -IL 15** | SEQ ID NO: 2 | SEQ ID NO: 4 | SEQ ID NO: 6 | SEQ ID NO: 18 | SEQ ID NO: 8 | SEQ ID NO: 32 | SEQ ID NO: 36 / SEQ ID NO: 42 |
| **pDE1-CYB5R3 -IL21** | SEQ ID NO: 2 | SEQ ID NO: 4 | SEQ ID NO: 6 | SEQ ID NO: 20 | SEQ ID NO: 8 | SEQ ID NO: 33 | SEQ ID NO: 36 / SEQ ID NO: 43 |
| **pDE1-CYB5R3 -IL37** | SEQ ID NO: 2 | SEQ ID NO: 4 | SEQ ID NO: 6 | SEQ ID NO: 22 | SEQ ID NO: 8 | SEQ ID NO: 34 | SEQ ID NO: 36 / SEQ ID NO: 44 |
| **pDE1-CYB5R3 - GMCSF** | SEQ ID NO: 2 | SEQ ID NO: 4 | SEQ ID NO: 6 | SEQ ID NO: 24 | SEQ ID NO: 8 | SEQ ID NO: 35 | SEQ ID NO: 36 / SEQ ID NO: 45 |

### Example 2: Confirmation of protein expression of dual expression vector

In order to confirm whether the dual expression vector constructed by the same method as in Example 1 can express an HIF-1α inhibitor and a cytokine normally, first, a HEK293T cell line was seeded in DMEM (containing 4.5 g/L D-glucose) supplemented with 10% fetal bovine serum (FBS) so as to become 4×10⁵ cells and cultured for 24 hours. Thereafter, the medium was removed, the cells were washed once with phosphate buffered saline (PBS), and then 2 mL of a serum-free OPTI-MEM medium was added thereto. Then, 3 µg of the dual expression vector and 0.1 µg of a pEGFP-C1 vector were transfected for 4 hours using Lipofectamine 2000, and then 2 mL of an OPTI-MEM medium supplemented with 20% fetal bovine serum was added, and the resulting mixture was incubated for 16 hours. Then, the existing medium was removed, a DMEM medium supplemented with 10% fetal bovine serum was added thereto, and the resulting mixture was further incubated for 24 hours.

In order to perform western blotting, the cell culture was centrifuged at 2,000xg at 4°C for 10 minutes, and the supernatant was transferred to a new tube. Then, after 200 µL of the supernatant was mixed with the same volume of 2X Laemmli sample buffer, the resulting mixture was boiled at 95°C for 5 minutes and cooled to prepare a media sample (growth media). Then, for cell samples (total cell extracts), cells were collected by scraping the plate surface with a cell lifter, then centrifuged at 3,000xg at 4°C for 5 minutes to remove the supernatant, 200 µL of NP-40 lysis buffer to which a protease inhibitor cocktail was added was added thereto, and the cells were disrupted using a sonicator. After the disrupted cells were mixed with the same volume of 2X Laemmli sample buffer, the resulting mixture was boiled at 95°C for 5 minutes and cooled to prepare a cell sample.

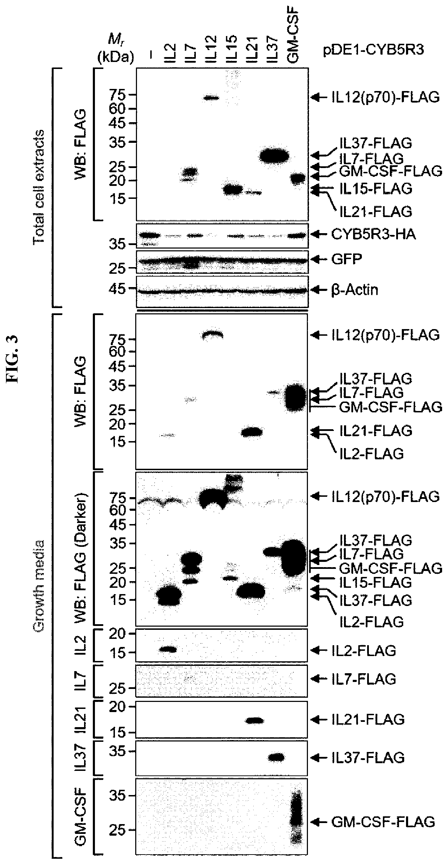

Western blotting was performed using the prepared cell sample and the media sample. The cell sample was subjected to western blotting using a mouse anti-FLAG M2 antibody, a mouse anti-HA antibody, a mouse anti-GFP antibody, and a mouse anti-β-actin antibody, and the media sample was subjected to western blotting using a mouse anti-FLAG M2 and specific antibodies capable of binding to each endogenous cytokine. All antibodies were purchased from Abcam, Sigma, and Cell Signaling Technology, respectively. The results are shown in FIG. 3.

As shown in FIG. 3, as a result of confirming the transfection efficiency in cell samples, it was confirmed that the transfection was normally induced because GFP was expressed at similar levels in all the samples by the GFP vector co-transfected as a control. Also, as a result of confirming the expression of cytokines using a FLAG antibody, it was confirmed that no cytokine was observed in the pDE1-CYB5R3 vector, and the cytokines to which FLAG inserted into each of the remaining vectors was bound were normally expressed. In addition, as a result of confirmation using an HA antibody, it was confirmed that the CYB5R3 protein to which HA was bound was normally expressed in all vectors.

Furthermore, as a result of confirmation using the FLAG antibody in the media sample, the cytokines to which FLAG was bound were normally expressed, and even in the result of confirmation using specific antibodies capable of binding to each cytokine, it was confirmed that the cytokines were normally expressed. In the case of GM-CSF-FLAG, a protein with a size of 20 kDa was mainly confirmed in cells, but a protein with a size of 25 to 35 kDa was confirmed in the media sample, and through this, it could also be confirmed that GM-CSF expressed by the dual expression vector was normally subjected to post-translational modification such as glycosylation. In the case of IL-2, looking at the results of detection with the FLAG antibody, it can be observed that IL-2 is weakly detected in cells, but is detected at high concentrations in the cell culture medium, indicating that most of the IL-2 produced in cells is rapidly secreted out of the cells with high efficiency.

### Example 3: Confirmation of anticancer effect of dual expression vector

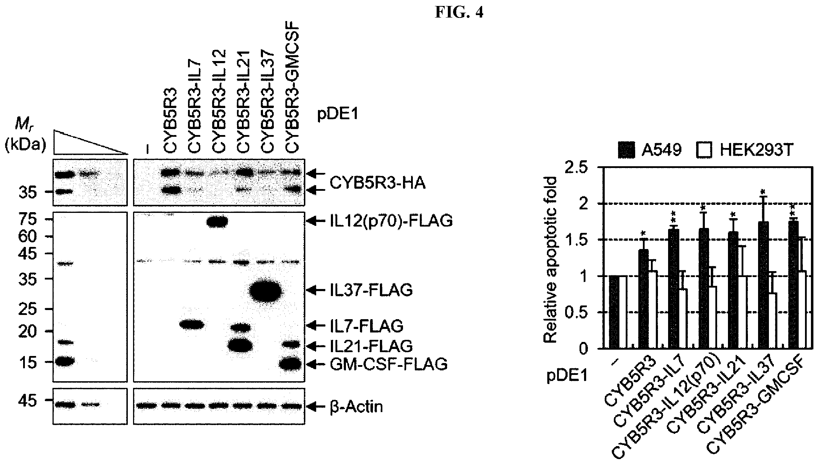

In order to confirm the anticancer effect of the dual expression vector constructed by the same method as in Example 1, an experiment was performed using the HEK293T cell line, which is a group of normal cells, and an A549 cell line, which is a group of cancer cells. More specifically, each cell line was seeded in DMEM (containing 4.5 g/L D-glucose) supplemented with 10% fetal bovine serum or RPMI1640 (containing 4.5 g/L D-glucose) supplemented with 10% fetal bovine serum so as to become 4 × 10⁵ cells, and cultured for 24 hours. Thereafter, the medium was removed, the cells were washed once with phosphate buffered saline (PBS), and then 2 mL of a serum-free OPTI-MEM medium was added thereto. Then, after 4 µg of the dual expression vector was transfected using the Lipofectamine 3000 and P3000 reagents for 4 hours, the medium was thoroughly removed, 3 mL of DMEM (containing 1 g/L D-glucose) supplemented with 10% fetal bovine serum or RPMI1640 (containing 2 g/L D-glucose) supplemented with 10% fetal bovine serum was added, and then the resulting mixture was incubated for 2 days. Two days later, the cell culture medium was transferred to a new tube, and the cells were washed with 3 mL of ice-cold PBS to thoroughly remove the medium, transferred to a new tube, and then 1 mL of Trypsin-EDTA was added thereto, and the resulting mixture was allowed to react at 37°C for 5 minutes. Then, after centrifugation at 200xg at 4°C for 5 minutes to remove the supernatant, the cell pellet was washed three times with ice-cold PBS, and the cells were suspended using 400 µL of 1X Annexin V Binding Buffer, and then the number of the cells was determined. Then, 1 × 10⁵ cells were put into a new tube, the tube was adjusted to a final volume of 200 µL using 1X Annexin V Binding Buffer, and then propidium iodide and Annexin V-APC were added thereto, and the resulting mixture was allowed to react in a dark room at room temperature for 20 minutes. Then, 300 µL of 1x Annexin V Binding Buffer was added, and the degree of propidium iodide staining and the degree of Annexin V-APC staining were quantitatively analyzed using a Novocyte Cell Analyzer, and the degree of apoptosis induction was confirmed using the same. For analysis, 10,000 cells were counted and the number of apoptotic cells among them was converted into a percentage. Then, the percentage of the apoptotic cells in pDE1-transfected cells used as a control was calculated as 1, and the relative percentages of the apoptotic cells of other dual expression vectors were relatively calculated. The remaining cells were subjected to western blotting in the same manner as in Example 2 to confirm whether the protein was normally expressed. Hereinafter, all experiments were performed in triplicate, and statistical significance was confirmed by a Student's test. * indicates P<0.05 and ** indicates P<0.01. The results are shown in FIG. 4.

As shown in FIG. 4, it was confirmed, by western blotting, that CYB5R3-HA and cytokine-FLAG proteins were normally expressed (left panel). Also, each vector did not induce apoptosis in normal cells, and through this, it was confirmed that the vector showed low cytotoxicity. In addition, it was confirmed that both the vector expressing the CYB5R3 protein alone and the dual expression vector expressing the cytokine along with the CYB5R3 protein remarkably increased apoptotic cells (right panel).

### Example 4: Confirmation of anticancer effect of dual expression vector using virus

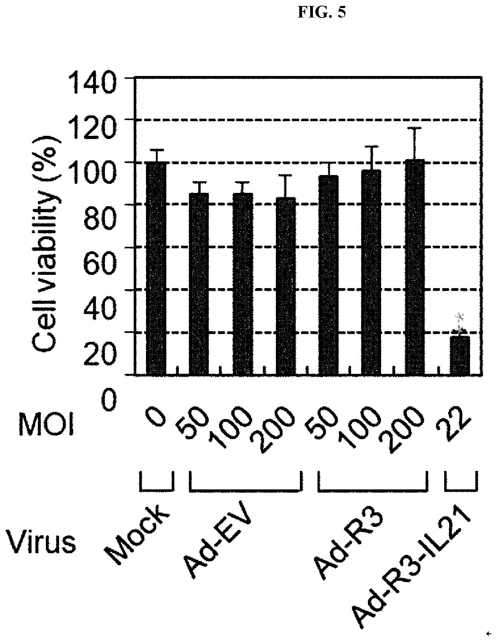

After the dual expression vector constructed by the same method as in Example 1 was injected into the virus, an anticancer effect was confirmed. More specifically, first, after the dual expression vector was injected into an adenovirus (TaKaRa) and an H1299 cell line, which is a lung cancer cell line, was treated with the virus so as to become a concentration of 22, 50, 100, or 200 MOI, respectively, the cells were cultured for 72 hours. Then, the cultured cells were washed twice with a phosphate buffer solution, and 100% methanol was added for stabilization at - 20°C for 15 minutes. Thereafter, the cells were washed three times with a phosphate buffer solution to completely remove the methanol, treated with 0.4% sulforhodamine B containing 1% acetic acid, allowed to react at room temperature for 30 minutes to stain the cells, and washed 4 times with 1% acetic acid to remove the residual staining solution. Then, finally, a 10 mM Tris solution was added to the cells, and the amount of remaining cells was measured by measuring absorbance at 565 nm to confirm the anticancer effect. The results are shown in FIG. 5.

As shown in FIG. 5, it was confirmed that, even in an anticancer effect experiment using the virus-injected vector, compared to Ad-R3 in which only the cyb5R3 gene was inserted, a remarkable anticancer effect was shown even upon treatment with a small MOI amount in the case of a dual expression vector in which both cyb5R3 and IL-21 genes were inserted. Through the results, it could be confirmed that an anticancer effect was shown even upon treatment with the dual expression vector of the present invention using a virus.

### Example 5: Confirmation of NK immune cell activation effect of dual expression vector

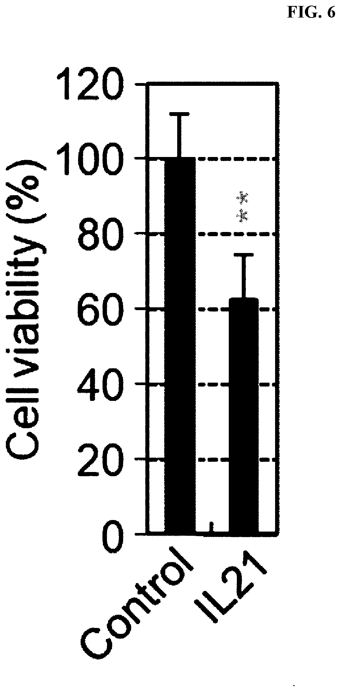

In order to confirm the NK immune cell activation effect of the dual expression vector constructed by the same method as in Example 1, first, the dual expression vector was injected into an adenovirus, and the HEK293T cell line was treated with the virus so as to become a concentration of 50 MOI and cultured for 72 hours to obtain a culture solution (supernatant). Then, the H1299 cell line and the NK92 cell line, which is a human NK cell line, were each treated with the same amount of the obtained culture solution and further cultured for 4 hours. As a control, a medium containing a cytokine secreted from a vector in which only the cyb5R3 gene was inserted was used. Then, the anticancer effect by NK immune cells was confirmed by staining the cells in the same manner as in Example 4 to measure the amount of remaining cells. The results are shown in FIG. 6.

As shown in FIG. 6, it was confirmed that IL-21 secreted from the dual expression vector increased the cancer cell-specific killing effect of NK cells compared to the control. Through the results, it could be confirmed that the anticancer cytokines expressed by the dual expression vector of the present invention increased the degree of activation of NK immune cells and increased the anticancer effect.

### Example 6: Confirmation of anticancer immune activation effect of dual expression vector

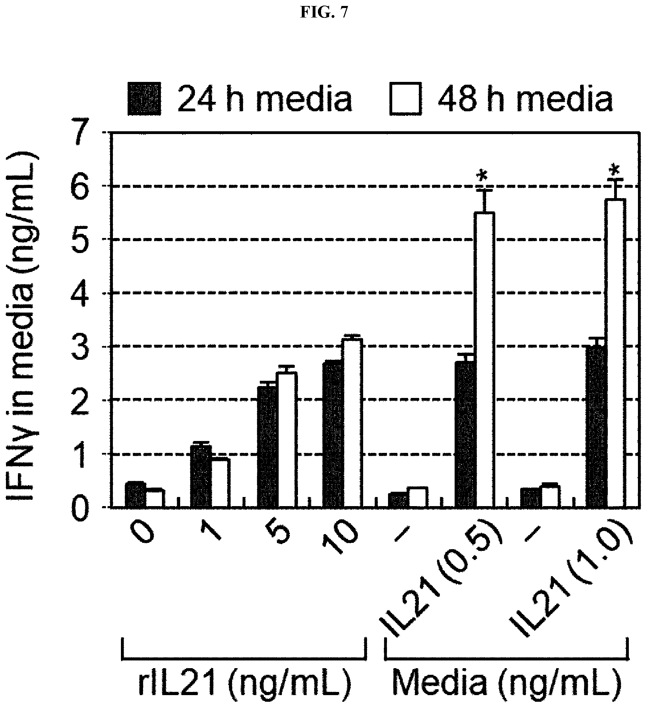

The anticancer immune activation effect of the dual expression vector constructed by the same method as in Example 1 was confirmed. More specifically, after the NK-92 cell line was treated with either 50 MOI of an adenovirus in which the dual expression vector was injected or human rII,21 (Thermo Fisher), the ability of IL21 to secrete IFN-gamma was confirmed at 24 and 48 hours using ELISA. Then, the secretion amount of IL21 expressed by the dual expression vector was also measured by performing ELISA using the cell culture solution. The results are shown in FIG. 7.

As shown in FIG. 7, it was confirmed that in the case of direct treatment with rIL21, up to 3 ng/mL of IFN-gamma was secreted up to 48 hours even in the case of treatment with 10 ng/mL rIL21, but in the case of treatment with the dual expression vector, 5 ng/mL or more IFN-gamma was secreted after 48 hours even though the same amount of IL21 was expressed. Through the results, it could be confirmed that when a dual expression vector in which the anticancer cytokine of the present invention and an HIF-1α inhibitor, which is a metabolic anticancer inhibitor, were both inserted, the immune activation effect of immune cells, such as NK cells, could be further remarkably increased compared to the anticancer cytokine treated alone.

Through the results, it could be confirmed that the dual expression vector of the present invention can simultaneously reduce side effects and improve the treatment effect due to the expression of protein capable of inhibiting HIF-1α and anticancer cytokine at the same time, through the protein capable of inhibiting HIF-1α, that is, a metabolic anticancer drug, can effectively inhibit the proliferation, migration, metastasis, and the like of cancer, and the anticancer cytokine, that is, a cancer immunotherapy drug, can induce cancer-specific immune response activating T cells, B cells, NK cells, and the like. In addition, even though the cells were treated with the same amount of the anticancer cytokine, the immune anticancer activation effect may be further improved upon expression while being inserted into a vector compared to the case of single treatment, so that it could be confirmed that, compared to the case of simple co-administration of the anticancer cytokine and the metabolic anticancer drug, the anticancer treatment effect can be more remarkably enhanced and can be effectively applied to the treatment of various types of cancers.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

Since the dual expression vector for simultaneously expressing an HIF-1α inhibitor and an anticancer cytokine according to the present invention simultaneously expresses a cancer immunotherapy drug and a metabolic anticancer drug, the dual expression vector not only can remarkably increase the cancer cure rate, but also can provide various treatment options of metabolic anticancer drugs and cancer immunotherapy drugs, and thus can be effectively applied to the treatment of various types of cancers without limitation.

## Claims

1. A pharmaceutical composition for preventing or treating cancer, comprising, as an active ingredient, a recombinant vector comprising a polynucleotide encoding a hypoxia-inducible factor-1α inhibitor, a polynucleotide encoding a 2A peptide, and a polynucleotide encoding an anticancer cytokine.

2. The pharmaceutical composition of claim 1, wherein the recombinant vector is in a form in which a polynucleotide sequence encoding a 2A peptide is inserted between a polynucleotide encoding a hypoxia-inducible factor-1α inhibitor and a polynucleotide encoding an anticancer cytokine.

3. The pharmaceutical composition of claim 1, wherein the hypoxia-inducible factor-1α inhibitor is cytochrome b5 reductase.

4. The pharmaceutical composition of claim 3, wherein the cytochrome b5 reductase comprises an amino acid sequence represented by SEQ ID NO: 1.

5. The pharmaceutical composition of claim 1, wherein the 2A peptide comprises an amino acid sequence represented by SEQ ID NO: 5.

6. The pharmaceutical composition of claim 1, wherein the anticancer cytokine is any one or more selected from the group consisting of interferon-alpha, interferon-beta, interferon-gamma, interleukin 2, interleukin 7, interleukin 12, interleukin 15, interleukin 18, interleukin 21, interleukin 37, and a granulocyte-macrophage colony-stimulating factor.

7. The pharmaceutical composition of claim 1, wherein the recombinant vector is linear DNA, plasma DNA, or a viral vector.

8. The pharmaceutical composition of claim 7, wherein the viral vector is any one or more selected from the group consisting of a retrovirus, an adenovirus, an adeno-associated virus, vaccinia virus, Myxoma virus, herpes simplex virus, and a lentivirus.

9. The pharmaceutical composition of claim 1, wherein the cancer is any one or more selected from the group consisting of melanoma, small cell lung cancer, non-small cell lung cancer, glioma, liver cancer, thyroid tumors, gastric cancer, prostate cancer, breast cancer, ovarian cancer, bladder cancer, lung cancer, colorectal cancer, breast cancer, prostate cancer, glioblastoma, endometrial cancer, renal cancer, colon cancer, esophageal carcinoma, head and neck cancer, mesothelioma, sarcomas, bile duct cancer, small bowel adenocarcinoma, pediatric malignant cancer, and epidermal cancer.

10. A food composition for preventing or improving cancer, comprising, as an active ingredient, a recombinant vector comprising a polynucleotide encoding a hypoxia-inducible factor-1α inhibitor, a polynucleotide encoding a 2A peptide, and a polynucleotide encoding an anticancer cytokine.

11. A method for preventing or treating cancer, the method comprising administering a composition comprising, as an active ingredient, a recombinant vector comprising a polynucleotide encoding a hypoxia-inducible factor-1α inhibitor, a polynucleotide encoding a 2A peptide, and a polynucleotide encoding an anticancer cytokine to a subject in need.

12. A use of a composition comprising, as an active ingredient, a recombinant vector comprising a polynucleotide encoding a hypoxia-inducible factor-1α inhibitor, a polynucleotide encoding a 2A peptide, and a polynucleotide encoding an anticancer cytokine for preventing or treating cancer.

13. A use of a recombinant vector comprising a polynucleotide encoding a hypoxia-inducible factor-1α inhibitor, a polynucleotide encoding a 2A peptide, and a polynucleotide encoding an anticancer cytokine for the manufacture of medicine for preventing or treating a cancer.
